# EUROPÄISCHE PATENTSCHRIFT

(11) **EP 0 471 883 B1**
(45) Veröffentlichungstag und Bekanntmachung des Hinweises auf die Patenterteilung: **02.11.1994**
(21) Anmeldenummer: 90116267.7
(22) Anmeldetag: 24.08.1990
(51) Int. Cl.: A61B 6/00, H05G 1/44

(54) **Röntgenbelichtungsautomat**
X-ray exposure automat
Régulateur automatique d'exposition à rayons X

(43) Veröffentlichungstag der Anmeldung: 26.02.1992
(73) Patentinhaber: SIEMENS AKTIENGESELLSCHAFT, 80333 München (DE)
(72) Erfinder: Aichinger, Horst, Dr. rer. nat., D-8510 Fürth (DE)

(56) Entgegenhaltungen:
- EP-A- 0 011 848
- FR-A- 2 477 826
- US-A- 4 097 741
- US-A- 4 189 645

## Beschreibung

In der Röntgentechnik, z.B. in der Mammographie, wird der Detektor für die Belichtungsautomatik generell hinter dem Film-Folien-System angeordnet. Dies führt bei den üblichen Röntgenbelichtungsautomaten zu einer Abhängigkeit der Belichtung von der Objektdicke. Ein automatischer Transparenzangleich ist möglich, wenn gemäß der DE-A-30 08 261 ein Detektor mit zwei Detektorelementen benutzt wird, deren auftreffende Strahlung unterschiedlich gefiltert wird und bei denen der Quotient der Signale der Detektorelemente gebildet wird. Dieser Quotient ist dann ein von der Transparenz abhängiges Signal, das zum Angleich des Belichtungsautomaten benutzt werden kann.

Trotz des automatischen Transparenzangleiches resultiert eine optimale Belichtung nur dann, wenn die Lage des Meßfeldes mit dem dichtesten Bereich der Mamma zur Deckung gebracht werden kann.

Der Erfindung liegt die Aufgabe zugrunde, einen Röntgenbelichtungsautomaten der eingangs genannten Art zu schaffen, bei dem die Meßfeldlage automatisch optimal gewählt wird.

Diese Aufgabe ist erfindungsgemäß dadurch gelöst, daß der Röntgenbelichtungsautomat mit zwei in Strahlenrichtung nach dem Untersuchungsraum und dem Bildaufnahme-System hintereinanderliegenden Strahlendetektoren versehen ist, von denen jeder aus einer Matrix von Detektorelementen gebildet ist, welche an einem Rechner zur Bestimmung des Quotienten der Signale von jeweils zwei hintereinanderliegenden Detektorelementen angeschlossen sind, wobei zur Auswahl des Meßfeldes für eine Röntgenaufnahme automatisch nur diejenigen Paare von Detektorelementen berücksichtigt werden, deren Quotient oberhalb einer vorbestimmten Schwelle liegt. Bei dem erfindungsgemäßen Röntgenbelichtungsautomaten werden diejenigen Detektorelemente, bei denen der Quotient der Ausgangssignale unter der vorgegebenen Schwelle liegt, die also hinter den weniger dichten Objektbereichen liegen, automatisch abgeschaltet. Das Meßfeld wird demgemäß automatisch hinter den dichtesten Bereich der Mamma gelegt. Auch Detektorelemente, die von Direktstrahlung getroffen werden, werden abgeschaltet.

Die Erfindung ist nachfolgend anhand eines in der Zeichnung dargestellten Ausführungsbeispieles näher erläutert. Es zeigen:
- Fig. 1: einen Röntgenbelichtungsautomaten nach der Erfindung,
- Fig. 2: einen Detektor des Röntgenbelichtungsautomaten gemäß Fig. 1, und
- Fig. 3: Kurven zur Erläuterung der Fig. 1 und 2.

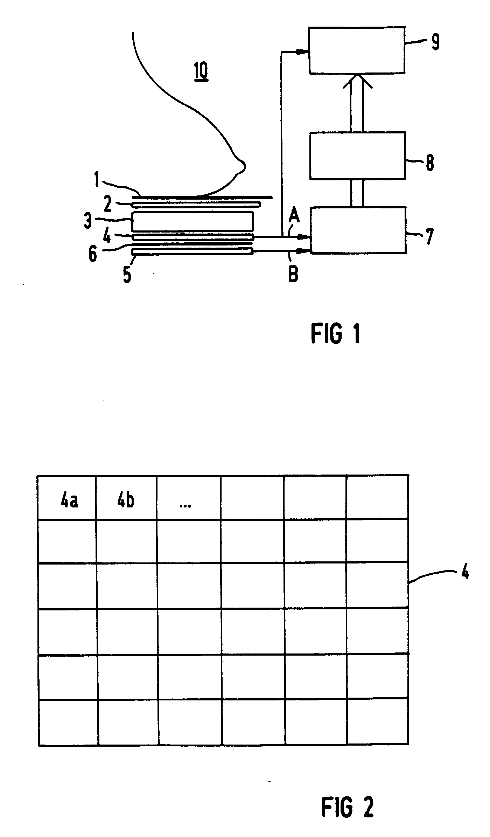

In der Fig. 1 ist ein für die Mammographie vorgesehener Röntgenbelichtungsautomat gezeigt, der hinter einer Auflageplatte 1, einem Streustrahlenraster 2 und einer Röntgenfilmkassette 3 zwei Detektoren 4, 5 aufweist, die durch ein Röntgenstrahlenfilter 6 voneinander getrennt sind. Die Detektoren 4, 5 sind an einem Rechner 7 angeschlossen, welcher über ein Korrekturglied 8 die Schaltstufe 9 des Belichtungsautomaten der jeweiligen Objekttransparenz entsprechend steuert.

Die Fig. 2 zeigt beispielsweise den Detektor 4. Dieser ist aus einer Matrix von Detektorelementen 4a, 4b usw. aufgebaut, die einzeln am Rechner 7 angeschlossen sind. Analog dazu ist der Detektor 5 aufgebaut.

Die Fläche der Detektoren 4, 5 erstreckt sich über das ganze Filmformat. Eine Größe von 3 cm x 4 cm für die Detektorelemente 4a, 4b usw. ist sinnvoll.

Jedes Detektorelement 4a, 4b usw. liefert während der Aufnahme ein der empfangenen Strahlungsintensität entsprechendes Ausgangssignal. Der Rechner 7 bildet für jedes Paar von Detektorelementen 4a, 4b usw., dessen Detektorelemente in Strahlenrichtung hintereinander und hinter dem Aufnahmeraum 10 liegen, den Quotienten. Dieser Quotient ist ein Maß für die jeweils davor liegende Objektdicke.

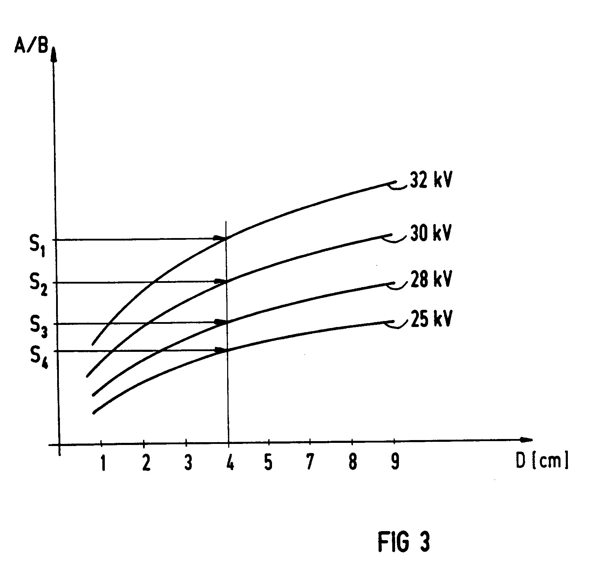

Die Fig. 3 zeigt die Abhängigkeit dieses Quotienten A/B von der Objektdicke D für vier verschiedene Röntgenröhrenspannungen. Mit der Objektdicke des jeweils vorgelagerten Objektbereiches steigt das Signal A/B bei einer bestimmten Röntgenröhrenspannung an. Es steigt natürlich auch in der Abhängigkeit von der Objektdichte, d.h. letztlich von der Transparenz an.

Durch Vorgabe einer Quotientenschwelle S1, S2, S3, S4 erfolgt eine automatische Meßfeldwahl. Nur diejenigen Detektorelemente 4a, 4b usw., die zu Paaren gehören, deren Signalquotient oberhalb der gewählten Schwelle S1 bis S4 liegt, werden für die Messung angewählt. Die anderen Detektorelemente 4a, 4b usw. werden abgeschaltet. So werden z.B. bei einer Röntgenröhrenspannung von 32 kV nur diejenigen Paare von Detektorelementen angewählt, bei denen der Quotient der Ausgangssignale oberhalb der Schwelle S1 liegt. Dies entspricht z.B. einer Dicke bzw. Transparenz entsprechend 4 cm Plexiglas. Auf diese Weise liegt das für die Belichtungsautomatik ausgewählte Meßfeld immer hinter dem dicken bzw. dichten Objektbereich, d.h. hinter dem Objektbereich mit der niedrigen Transparenz.

Nachdem während einer laufenden Aufnahme die Meßfeldwahl abgeschlossen ist, wird das Summensignal der Detektorelemente 4a, 4b usw. des oberen Detektors 4 im ausgewählten Meßfeld allein zum Abschalten der Aufnahme herangezogen. Die Abschaltung erfolgt durch die Schaltstufe 9, die entsprechend angesteuert wird. Der Mittelwert der Quotientensignale im ausgewählten Meßfeld wird zur Korrektur des Sollwertes der Abschaltdosis der Schaltstufe 9 verwendet.

## Patentansprüche

1. Röntgenbelichtungsautomat mit zwei in Strahlenrichtung nach dem Untersuchungsraum (10) und dem Bildaufnahme-System (3) hintereinanderliegenden Strahlendetektoren (4, 5), von denen jeder aus einer Matrix von Detektorelementen (4a, 4b usw.) gebildet ist, welche an einem Rechner (7) zur Bestimmung des Quotienten der Signale von jeweils zwei hintereinanderliegenden Detektorelementen (4a, 4b usw.) angeschlossen sind, wobei zur Auswahl des Meßfeldes für eine Röntgenaufnahme automatisch diejenigen Paare von Detektorelementen (4a, 4b usw.) berücksichtigt werden, deren Quotient oberhalb einer vorbestimmten Schwelle liegt.

## Claims

1. Automatic X-ray exposure unit having two radiation detectors (4, 5) which are arranged in series in the direction of radiation after the examination space (10) and the image-receiving system (3) and each of which is formed from a matrix of detector elements (4a, 4b etc.) which are connected to a computer (7) for determining the quotient of the signals from, in each case, two detector elements (4a, 4b etc.) which are arranged in series, wherein those pairs of detector elements (4a, 4b etc.) having a quotient which lies above a predetermined threshold are automatically taken into consideration for the selection of the measuring field for an X-ray exposure.

## Revendications

1. Automate d'exposition aux rayons X comportant deux détecteurs de rayonnement (4,5) disposés l'un derrière l'autre dans la direction du rayonnement, en aval de l'espace d'examen (10) et du système (3) d'enregistrement d'images, et dont chacun est formé par une matrice d'éléments de détection (4a, 4b, etc.), qui sont raccordés à un calculateur (7) servant à déterminer le quotient des signaux de respectivement deux éléments de détection (4a,4b, etc.) disposés l'un derrière l'autre, les couples d'éléments de détection (4a, 4b), etc., dont le quotient est supérieur à un seuil prédéterminé, étant pris en compte automatiquement pour la sélection du champ de mesure pour une radiographie.
